# EUROPEAN PATENT SPECIFICATION

(11) **EP 2 665 700 B1**
(45) Date of publication and mention of the grant of the patent: **13.01.2016**
(21) Application number: 12736785.2
(22) Date of filing: 18.01.2012
(51) Int. Cl.: C07C 211/32, C07C 209/84, C07C 209/68

(54) **ONE-POT PREPARATION OF CYCLOBENZAPRINE HYDROCHLORIDE**
EIN-TOPF-PRÄPARAT AUS CYCLOBENZAPRIN-HYDROCHLORID
PRÉPARATION MONOTOPE D'HYDROCHLORURE DE CYCLOBENZAPRINE

(30) Priority: 20.01.2011 IN MM01712011
(43) Date of publication of application: 27.11.2013
(73) Proprietor: Harman Finochem Limited, Mumbai 400 098, Maharashtra (IN)
(72) Inventor: JAIN, Kirti Prakash, Rajashtan (IN); KHAN, Afzal Yousuf, Maharashtra (IN); MINHAS, Gurpreet Singh, Maharashtra (IN); MINHAS, Harpreet Singh, Maharashtra (IN)
(74) Representative: Isarpatent
(86) International application number: PCT/IN2012/000046
(87) International publication number: WO 2012/098563

(56) References cited:
- CN-A- 101 260 046
- CN-A- 101 260 046
- US-A- 3 454 643
- US-A- 3 454 643

## Description

### FIELD OF INVENTION:

The present invention relates to a cost effective one-pot process for the preparation of cyclobenzaprine hydrochloride suitable for pharmaceutical use.

### BACKGROUND OF THE INVENTION:

Cyclobenzaprine hydrochloride, chemically known as 5-(3-dimethylaminopropylidene)-dibenzo (a,e) cycloheptatriene hydrochloride (Formula I), 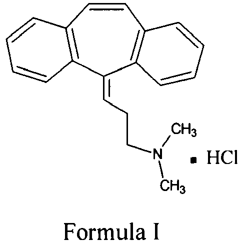 is a commonly prescribed tricyclic amine having muscle relaxant pharmaceutical activity. After sustaining an injury, muscle spasms may occur to stabilize the affected body part and prevent further damage. Cyclobenzaprine hydrochloride is used to treat such muscle spasm associated with acute, painful musculoskeletal conditions.

Few multistep processes for the preparation of this tricyclic amine are already available in the literature which involves isolation and purification of intermediate compounds. The conventional route of synthesis as reported in US3454643, ES8201950 includes preparation of Grignard reagent (GR) of 3-dimethylaminopropyl chloride in a first step, reacting with 5-dibenzosuberenone (FormulaII) in a second step. The reaction mass was extracted with benzene, solid obtained was recrystallized from alcohol to produce 5-hydroxy intermediate (Formula III) and further dehydrated in third step using acetyl chloride or acetic anhydride in presence of chloroform as a solvent medium followed by purging HCl gas to produce hydrochloride salt (Formula I).

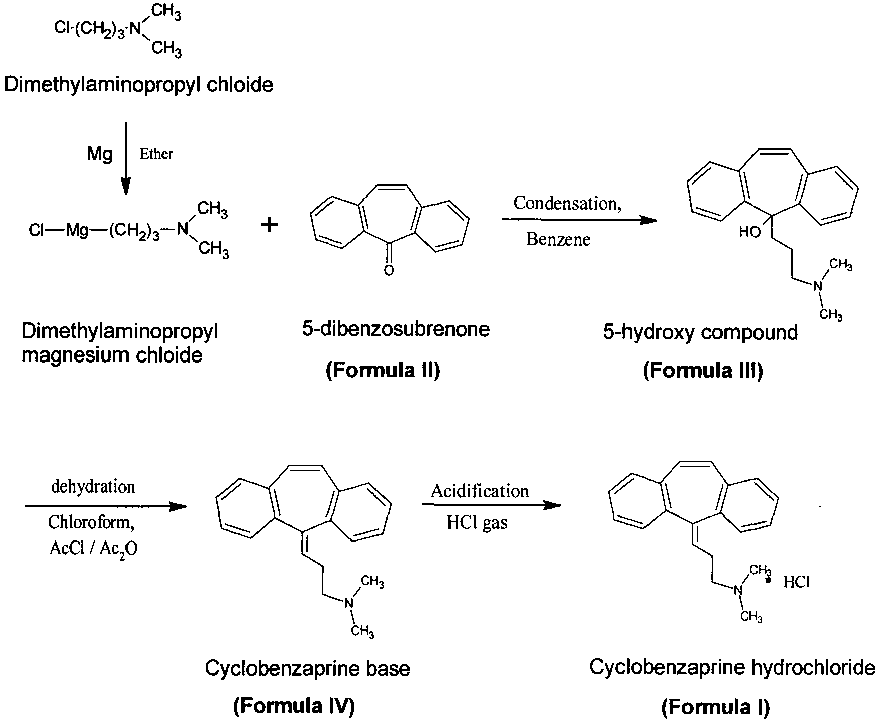

The multistep synthesis is cumbersome and use of hazardous solvents and reagents like chloroform, benzene and acetyl chloride etc are not recommended for the preparation of pharmaceutical substances.

J. Org. Chem. Vol. 27, 230-240 (1961**)** also portrayed similar procedure for the synthesis of cyclobenzaprine hydrochloride, wherein 5-hydroxy compound of formula III was isolated and recrystallized before dehydration reaction.

Synthetic Comm. 11 (3), 241-246 (1981**)** described a process which involves isolation and purification of the intermediate at magnesium -complex stage. Hydrolysis of the isolated complex afforded desired tricyclic amine.

GB858186 and GB858187 jointly described a process which comprises preparation of 5-hydroxy compound (Formula III) and subsequent conversion of the same to cyclobenzaprine hydrochloride. However the overall yield reported is significantly low.

In a different approach, a high temperature dehydrogenation of amitriptyline base resulting **in** formation of cyclobenzaprine hydrochloride is reported in Indian patent application 387/CHE/2005.

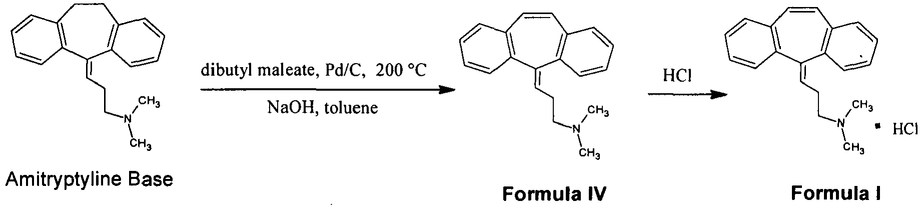

It has been observed that most of prior art processes are having multiple steps wherein isolation and purification of various intermediate stages are necessary, thus accountable for low yield of final product. Moreover the use of expensive and hazardous reagents or solvents makes the process uneconomical for the commercial production.

### Objects of the invention:

The main object of the present invention is to provide a convenient one-pot process for the preparation of cyclobenzaprine hydrochloride without isolation and purification of intermediate compounds.

Another object of the invention is to provide a cost effective procedure for the preparation of cyclobenzaprine hydrochloride in a high yield and high purity for pharmaceutical use. Another object of the invention is to provide a procedure for preparing cyclobenzaprine hydrochloride that overcomes the drawbacks of prior art processes.

### SUMMARY OF THE INVENTION:

In accordance with above objects, the present invention discloses a cost effective one-pot process for the preparation of cyclobenzaprine hydrochloride (Formula I) without isolation and purification of the intermediate compounds.

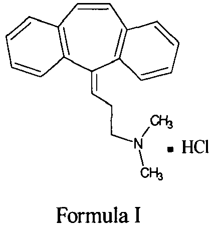

In an aspect of the invention, process for the preparation of cyclobenzaprine hydrochloride comprises reaction of 5-dibenzosuberenone (Formula II) with a Grignard reagent derived from 3- dimethylaminopropyl chloride, at a temperature of 0-15°C. The reaction mass undergoes hydrolysis and dehydration reaction without isolation of intermediate compound in presence of aq. HCl solution at a temperature of 50°- 100°C for a period of 1-6 hrs. After completion of the reaction, the reaction mass is neutralized and the product is extracted in an organic solvent which is further converted in to its hydrochloride salt (Formula I).

### DETAILED DESCRIPTION OF THE INVENTION:

The present invention provides a one-pot process for the preparation of cyclobenzaprine hydrochloride (Formula I).

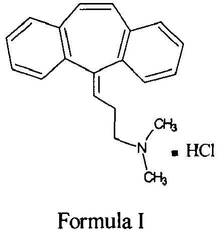

In a preferred embodiment of the invention, the one-pot process comprises the steps of-
a) preparing dimethylaminopropyl magnesium chloride (Grignard Reagent) by reacting 3-dimethylaminopropyl chloride and magnesium metal in presence of tetrahydrofuran;
b) reacting 5-dibenzosuberenone (Formula II) with Grignard Reagent at temperature 0-15°C.
c) hydrolyzing the reaction mass followed by dehydration without isolating the intermediate 5-hydroxy compound of Formula III in presence of 15-25% w/v aqueous hydrochloride solution at a temperature of 50°-100°C preferably at 70-80°C for period of 1-6 hrs preferably for 2-3hrs.; 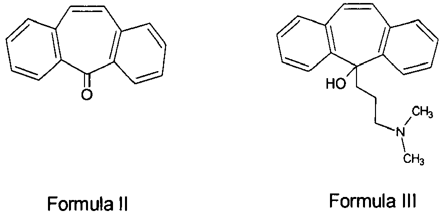
d) neutralizing the reaction mass with aqueous alkali solution followed by extraction. The removal of solvent under reduced pressure forms cyclobenzaprine base (Formula IV); 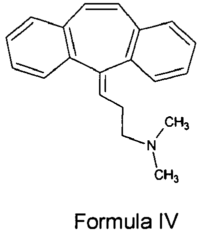
e) acidifying the compound of Formula IV with alcoholic hydrochloride solution such as IPA.HCl at a lower temperature of 0-10°C to give cyclobenzaprine hydrochloride (Formula I).

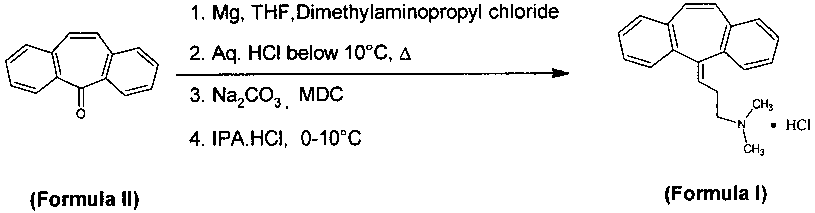

The aqueous alkali solution used for neutralization in step(d) is selected from group of sodium carbonate, potassium carbonate, sodium hydroxide and potassium hydroxide preferably aqueous sodium carbonate solution and the organic solvent used for extraction is selected from methylene dichloride, toluene and ethyl acetate, more preferably methylene dichloride.

In a single vessel, 5-dibenzosuberenone (Formula II) is reacted with dimethylaminopropyl magnesium chloride at a temperature 0-15°C for 30-90 min. The reaction mass undergoes hydrolysis and dehydration reaction in presence of 15-25% w/v aqueous hydrochloride solution by heating at a temperature about 70-80°C for 2-3 hrs. After completion of the reaction, the reaction mass is neutralized by using aqueous Na₂CO₃ solution and the product is extracted with methylene dichloride. After the complete removal of solvent, the oily mass is dissolved in isopropyl alcohol and the mixture is acidified by slow addition of IPA.HCl solution at 0-10°C with continuous stirring for 2-3 hrs for complete precipitation. The precipitate is filtered, recrystallized from isopropyl alcohol and dried to obtain cyclobenzaprine hydrochloride (Formula I) in high yield (70-80 %) and having purity above 99.90% (by HPLC).

The present invention has many advantages over the procedures previously disclosed in the art,
- The process allows the final product to be produced in a higher yield and purity by employing a one-pot process for the preparation of cyclobenzaprine hydrochloride by minimizing the reaction steps, thus avoiding the need for time consuming and cost prohibiting isolation of intermediates.
- Use of non hazardous solvents, reagents and reaction conditions eliminates the potential risk of using chemicals such as chloroform, benzene, acetic anhydride and acetyl chloride which are not acceptable according to the health standards.
- It can be readily employed at commercial scale suitable for pharmaceutical use.

The following example is given by way of illustration of the present invention and should not be construed to limit the scope of the present invention.

### EXAMPLE:

In a reaction vessel, THF (110ml), magnesium turnings 20gm (0.823mole) were charged and the mixture was warmed to 45-55°C for 20 min. A solution of 100gm (0.823mole) of 3-dimethylaminopropyl chloride prepared in 110ml THF was added dropwise to the reaction mixture by controlling the reflux generated due to reaction initiation and maintained for 2hrs. The formed Grignard reagent was then cooled to 0-5°C and a solution of 100gm (0.485mole) 5-dibenzosuberenone prepared in 220ml THF was charged to the reaction mass at temperature below 10°C. The reaction mass was stirred for 45 min at temperature 10-15°C. The absence of 5-dibenzosuberenone was checked by TLC and 770ml of 20% aq. HCl was charged to the reaction mass at a temperature below 10°C. The reaction mass was then heated to 70-80°C for 3 hrs. The acidic mass was neutralized by using aqueous Na₂CO₃ solution and extracted with 900ml methylene dichloride. The solvent was removed completely under reduced pressure and oil thus formed was dissolved in 450ml IPA and acidified with 240 ml of 20% IPA .HCl solution and stirred for 2 hrs at 0-5°C for complete precipitation. The precipitate is filtered, recrystallized from IPA (800 ml) and dried to obtain 118 gm (78%) white crystalline cyclobenzaprine hydrochloride with purity 99.93% by HPLC.

## Claims

1. A cost effective one-pot process for the preparation of cyclobenzaprine hydrochloride (Formula I) comprising steps of,
a. preparing Grignard reagent by reacting 3-dimethylaminopropyl chloride and magnesium metal in tetrahydrofuran;
b. reacting 5-dibenzosuberenone(Formula II) with Grignard reagent to obtain reaction mass;
c. hydrolyzing the reaction mass followed by dehydration without isolating the intermediate 5-hydroxy compound of formula III in presence of 15-25% w/v aqueous hydrochloric acid;
d. neutralizing the reaction mass with aqueous alkali solution followed by extraction to form cyclobenzaprine base (Formula IV); and
e. acidifying the base (Formula IV) in to its hydrochloride salt (Formula I).

2. The process according to claim 1, wherein the dehydration reaction in step c) is carried out at a temperature range of 50-100°C preferably at 70-80°C.

3. The process according to claim 1, wherein the dehydration reaction in step c) is maintained for 1-6 hrs, preferably for 2-3 hrs.

4. The process according to claim 1, wherein aqueous alkali solution used for neutralization in step d) is selected from group of sodium carbonate, potassium carbonate, sodium hydroxide and potassium hydroxide, preferably sodium carbonate.

5. The process according to claim 1, wherein the solvent used for extraction in step d) is selected from methylene dichloride, toluene and ethyl acetate, more preferably methylene dichloride.

6. A process according to claim 1, wherein the acidification in step e) is carried out using IPA.HCl solution at a temperature below 10°C.

7. The process as claimed in any of the preceding claims, wherein purity of cyclobenzaprine hydrochloride produced is above 99.90% by HPLC.

## Patentansprüche

1. Kostengünstiges Verfahren zur Herstellung von Cyclobenzaprinhydrochlorid (Formel 1) in einem Behältnis umfassend die folgenden Schritte:
a. Herstellung eines Grignard-Reagens durch das Umsetzen von 3-Dimethylaminopropylchlorid und Magnesium-Metall in Tetrahydrofuran;
b. Umsetzen von 5-Dibenzosuberenon (Formel II) mit einem Grignard-Reagens, um die Reaktionsmasse zu erhalten;
c. Hydrolysieren der Reaktionsmasse gefolgt von Dehydration ohne Isolieren der Zwischen-5-Hydroxy-Verbindung der Formel III in der Gegenwart von 15-25% w/v wässriger Hydrochlorsäure;
d. Neutralisieren der Reaktionsmasse mit einer wässrigen Alkalilösung, gefolgt durch die Extraktion, um eine Cyclobenzaprinbase (Formel IV) zu bilden; und
e. Ansäuern der Base (Formel IV) in ihr Hydrochloridsalz (Formel I).

2. Verfahren nach Anspruch 1, wobei die Dehydrierungsreaktion in Schritt c) in einem Temperaturbereich von 50 bis 100 °C durchgeführt wird, bevorzugt bei 70 bis 80 °C.

3. Verfahren nach Anspruch 1, wobei die Dehydrierungsreaktion in Schritt c) für 1 bis 6 Stunden beibehalten wird, bevorzugt für 2 bis 3 Stunden.

4. Verfahren nach Anspruch 1, wobei die wässrige Alkalilösung, die für die Neutralisierung in Schritt d) verwendet wird, ausgewählt ist aus der Gruppe bestehend aus Natriumcarbonat, Kaliumcarbonat, Natriumhydroxid und Kaliumhydroxid, bevorzugt Kaliumcarbonat.

5. Verfahren nach Anspruch 1, wobei das Lösungsmittel, das für die Extraktion in Schritt d) verwendet wird, ausgewählt ist aus Methylendichlorid, Toluol- und Ethylacetat, besonders bevorzugt Methylendichlorid.

6. Verfahren nach Anspruch 1, wobei die Veresterung in Schritt e) unter Verwendung einer IPA.HCl-Lösung bei einer Temperatur unter 10 °C durchgeführt wird.

7. Verfahren nach einem der vorangehenden Ansprüche, wobei die Reinheit des durch HPLC erzeugten Cyclobenzaprinhydrochlorids über 99,90 % beträgt.

## Revendications

1. Procédé économiquement rentable à réacteur unique pour la préparation du chlorhydrate de cyclobenzaprine (Formule I) comprenant les étapes de :
a. préparation du réactif de Grignard par la réaction du chlorure de 3-diméthylaminopropyle et du magnésium métal dans le tétrahydrofurane ;
b. réaction de la 5-dibenzosubérénone (Formule II) avec le réactif de Grignard pour obtenir la masse réactionnelle ;
c. hydrolyse de la masse réactionnelle suivie d'une déshydratation sans isolation du composé 5-hydroxy intermédiaire de formule III en présence de 15 à 25 % en poids/volume d'acide chlorhydrique aqueux ;
d. neutralisation de la masse réactionnelle par une solution alcaline aqueuse suivie d'une extraction pour former la cyclobenzaprine base (Formule IV) ; et
e. acidification de la base (Formule IV) sous forme de son sel chlorhydrate (Formule I).

2. Procédé selon la revendication 1, dans lequel la réaction de déshydratation de l'étape c) est réalisée dans une gamme de température entre 50 et 100 °C, de préférence de 70 à 80 °C.

3. Procédé selon la revendication 1, dans lequel la réaction de déshydratation de l'étape c) est entretenue pendant 1 à 6 heures, de préférence, pendant 2 à 3 heures.

4. Procédé selon la revendication 1, dans lequel la solution alcaline aqueuse utilisée pour la neutralisation de l'étape d) est choisie dans le groupe formé par le carbonate de sodium, le carbonate de potassium, l'hydroxyde de sodium et l'hydroxyde de potassium, de préférence, le carbonate de sodium.

5. Procédé selon la revendication 1, dans lequel le solvant utilisé pour l'extraction de l'étape d) est choisi parmi le dichlorure de méthylène, le toluène et l'acétate d'éthyle, de préférence, le dichlorure de méthylène.

6. Procédé selon la revendication 1, dans lequel l'acidification de l'étape e) est réalisée en utilisant une solution d'IPA, HCl à une température inférieure à 10 °C.

7. Procédé tel que revendiqué dans l'une quelconque des revendications précédentes, dans lequel la pureté par HPLC du chlorhydrate de cyclobenzaprine produit est supérieure à 99,90 %.
